(19) Europäisches Patentamt
European Patent Office
Office européen des brevets

(11) Publication number: 0 195 534 B1

(12) **EUROPEAN PATENT SPECIFICATION**

(45) Date of publication of the patent specification: **31.05.89**

(51) Int. Cl.[4]: **C 07 C 11/06,** C 07 C 7/148

(21) Application number: **86301298.5**

(22) Date of filling: **24.02.86**

(54) **Process for removing carbonyl sulphide from liquid propylene.**

(30) Priority: **04.03.85 US 707645**

(43) Date of publication of application: **24.09.86 Bulletin 86/39**

(45) Publication of the grant of the patent: **31.05.89 Bulletin 89/22**

(84) Designated Contracting States: **BE DE FR GB IT NL**

(56) References cited: **US-A-4 491 516**

(73) Proprietor: **EXXON CHEMICAL PATENTS INC., 200 Park Avenue, Florham Park New Jersey 07932 (US)**

(72) Inventor: **Deason, Douglas Louis, 1046 Chippenham, Baton Rouge Louisiana 70808 (US)**
Inventor: **Mohundro, Edgar Lucian, Esso Chemie GmbH, OTD Europe Postfach 600104, D-5000 Köln 60 (DE)**
Inventor: **Filupeit, Walter Frederick, 16430 Havenhurst Drive, Houston Texas 77059 (US)**

(74) Representative: **Dew, Melvyn John, Esso Chemical Ltd. Esso Chemical Research Centre P.O. Box 1, Abingdon Oxfordshire, OX13 6BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension)



LIBER, STOCKHOLM 1989

**Description**

This invention relates to a process for the removal of carbonyl sulfide, COS, from a liquid propylene feedstream. More particularly, this invention relates to an activated alumina catalyzed hydrolysis process for COS removal from a liquid propylene feedstream containing substantial quantities of water.

Recently developed catalysts for the production of polypropylene require minimization of the content of sulfurous impurities present in the propylene which is introduced into the polypropylene manufacturing process. Current propylene specifications particularly require that the COS content not be in excess of about 50 wppb (weight parts per billion).

The prior art recognizes the problem and presents a number of techniques, including the use of hydrolysis, to remove COS from liquid propylene feedstreams. The COS hydrolysis reaction is represented by the equation:

$$COS + H_2O \rightarrow CO_2 + H_2S$$

Prior art disclosures dealing with COS hydrolysis techniques for liquid hydrocarbon feedstreams are represented by U.S. Patent 3 265 757, issued August 9, 1966 to Frevel et al.; U.S. Patent 4 444 987, issued April 24, 1984 to Brownell et al.; U.S. Patent 4 455 446, issued June 19, 1984 to Brownell et al.; and U.S. Patent 4 491 516, issued January 1, 1985 to Polleck et al.

Frevel et al. in U.S. Patent 3 265 757 disclose the use of a particular modified alumina catalyst which is treated so as to contain 0.5 to 3.0 % by weight of sodium or potassium atoms affixed thereto. In the hydrolysis reaction using this catalyst, the amount of water used is in the range of 0.3 times the COS wppm concentration up to 12T wppm (weight parts per million), T being the process temperature in degrees Centrigrade. The catalyst life is said to be indefinite and the finished product stream is said to contain less than 1 wppm of COS.

Brownell et al. in U.S. Patents 4 444 987 and 4 455 446 use a catalyst comprising platinum sulfide on alumina and require an amount of water such that the water to COS mole ratio is at least 2 : 1. Product streams containing less than 50 wppb are reported. Byproducts $CO_2$ and $H_2S$ are removed using a distillation procedure and catalyst is regenerated by dissolving polypropylene from the catalyst surface using a suitable solvent.

Polleck et al. in U.S. Patent 4 491 516 disclose the use of alumina as a catalyst for COS removal via hydrolysis of liquid propylene streams and require that the water content relative to COS be maintained at a defined critical ratio, which is said to be a water to COS mole ratio between 1 : 1 and 1 : 10, the preferred range being 1.5 : 1 to 6 : 1. If 30 % saturation provides a lesser amount then it is the permissible upper limit in water. Water is added periodically in the process to maintain the critical ratio, and Polleck et al. disclose that the use of excess water will inhibit the COS hydrolysis reaction. Polleck et al.'s process is said to provide very low levels of COS content, such as below 1 wppm and sometimes to levels below 100 wppb; for example, 50 wppb.

The present invention is considered distinguished from the foregoing references for the following reasons: (a) the process of the present invention uses unmodified activated alumina catalyst; i.e., the catalyst consists of only activated alumina and the invention expressly excludes catalysts other than activated alumina; (b) the water content of the propylene feedstream used in the present invention is substantially in excess of that considered permissible by the prior art; (c) periodic regeneration of the activated alumina catalyst is essential to maintain efficient hydrolysis; and (d) removal of byproducts, $H_2O$ and additional COS is accomplished through use of one or more supplemental reactors which also contain activated alumina.

In accordance with the present invention, there has been discovered a process for removing carbonyl sulfide from a liquid propylene feedstream to produce a propylene product containing less than 50 wppb carbonyl sulfide and being substantially free of $CO_2$, $H_2S$ and $H_2S$ which comprises treating a liquid propylene feedstream containing water, the treatment comprising the steps of: (a) conducting hydrolysis of the carbonyl sulfide in the liquid propylene feedstream by passing the feedstream over activated alumina in a hydrolysis reactor at a liquid hourly space velocity of 1 - 10 cubic metre of feedstream per cubic metre of activated alumina and at a temperature of 15° to 93°C (60° to 200°F), the amount of water in the feedstream undergoing hydrolysis being such that the molar ratio of water to carbonyl sulfide is at least 15 to 1; (b) continuing step (a) until the carbonyl sulfide content of the feedstream after hydrolysis is more than about 500 wppb, and in such event, discontinuing hydrolysis by removing the hydrolysis reactor from service and regenerating the catalyst, said regeneration being conducted by passing an inert gas at 204 - 260°C (400 - 500°F) through the catalyst for a period of 10 to 30 hours and thereafter resuming hydrolysis; and (c) removing $CO_2$, $H_2O$ and $H_2S$ by adsorption and additional COS by supplemental hydrolysis and adsorption by passing the feedstream through one or more secondary reactors containing activated alumina operated at ambient temperature whereby a liquid propylene containing less than 50 wppb COS and being substantially free of $CO_2$, $H_2O$ and $H_2S$ is obtained.

In the present invention, the feedstream to be treated contains substantial amounts of water and the amount of water may be permitted to vary widely. Thus, the present invention does not require careful control or maintenance of a critical $H_2O$ to COS ratio in order to have a process effective in reducing the COS content. Effective results have been achieved utilizing feedstreams containing water in an amount to provide a mole ratio of 50 to 600 moles of water per mole of COS, and preferably the water content will be 50 to 300 moles of water per mole of COS. The feedstream may contain water up to 100 % saturation.

The process of the invention is operated by passing the wet feedstream into the hydrolysis reactor. The feedstream is maintained at a pressure to keep it in liquid form such as 3447kPa (500 psig). The hydrolysis reactor is maintained at 15° to 93°C (60°F to 200°F), preferably 35° to 43°C (95°F to 110°F). The LHSV (liquid hourly space velocity) is 1 - 10 cubic metre per hour of feedstream per cubic metre of alumina, preferably 2.5 to 4. Downstream of the hydrolysis reactor are positioned in series one or more, preferably two, secondary supplemental reactors which contain activated alumina and which are maintained at ambient temperatures and which function to remove by adsorption water, the hydrolysis byproducts $CO_2$ and $H_2S$ from the feedstream, as well as serving to reduce the COS content to 50 wppb in the event the feedstream exiting the hydrolysis reactor contains from 50 to 500 wppb. The secondary reactors will remove COS by adsorption and by hydrolysis.

Activated aluminas useful in the present invention are those commercially available and known to be effective in catalyzing the COS hydrolysis reaction. Generally such activated aluminas have a high surface area; i.e., 50 - 500 $M^2/g$, preferably 250 - 400 $M^2/g$, and will contain less than 0.4 % of oxides of sodium, silicon or iron. These are normally supplied in spherical pellets of 3 - 8 U.S. Standard mesh and are used in the present invention in unmodified form.

Hydrolysis is observed by continuously monitoring the COS content of the liquid propylene product. When the analysis shows a COS content of the feedstream leaving the hydrolysis reactor significantly above 500 wppb, at that point the hydrolysis reactor is removed from service and the catalyst is regenerated.

In accordance with the invention, it has been found highly effective to regenerate the catalyst by passing through the hydrolysis reactor an inert gas at a temperature of of about 232°C (450°F), for 10 - 30 hours, preferably 16 - 18 hours. Preferably a hydrocarbon-containing gas, such as methane, natural gas or a demethanizer tail gas is used, but inert gases such as $H_2$ or $N_2$ are also considered suitable. The term inert gas as used herein refers to a gas which does not polymerize upon contact with alumina or otherwise form a reaction product with alumina. Gases containing substantial amounts of polymerizable olefins should therefore be avoided. Upon completion of the regeneration, the hydrolysis process in that reactor may be resumed.

It is within the scope of the present invention to employ two hydrolysis reactors alternatively; however, the loss in production time due to regeneration has been found not to be economically disadvantageous. Typically, regeneration is required for only 16 - 18 hours per 15 - 20 day period of hydrolysis reactor operation.

The supplemental secondary reactors for purifying and finishing the product also contain activated alumina and are permitted to operate at ambient temperature; i.e., at the temperature of the incoming stream from the hydrolysis reactor. These reactors remove by adsorption any remaining water as well as the byproducts of the hydrolysis reaction, $CO_2$ and $H_2S$. The final product is substantially free of $H_2O$, $CO_2$ and $H_2S$; i.e., it contains only trace amounts of these materials. These reactors also serve to reduce the COS content to 50 wppb or lower by adsorption or by a supplemental hydrolysis. Preferably, two secondary reactors are employed and they are generally of the same size and contain the same amount of activated alumina as the primary hydrolysis reactor.

The invention is further illustrated by the following example.

**Example**

The data listed below in Table I show operation of the hydrolysis reactor of the present invention utilizing a bed of 1.83 m (6 foot) diameter and a packed bed length of 4.57 m (15 feet) containing a total of 10.8 $m^3$ (380 cubic feet) of activated alumina. The alumina has a surface area of 350 $m^2/g$ and is in the form of spherical pellets. The data below are typical of actual performance. This data is presented to illustrate the wide variations permitted in water content while still obtaining a consistent COS content in the desirable area of about 50 wppb.

After a period of 15 - 20 days, the COS content of the stream leaving the hydrolysis reactor will rise to 400 - 500 wppb, at which point regeneration will be conducted using demethanizer tail gas at 232°C (450°F) for 16 - 18 hours.

**Table I**
**Hydrolysis Data**

| LHSV $m^3$/Hour/$m^3$ Alumina | Temperature, °C (°F) | Mole Ratio Water/COS | COS in Feed, wppm | % COS Conversion | wppb COS in Product |
|---|---|---|---|---|---|
| 3.9 | 42.2 (108) | 107 | 4.5 | 98.9 | 49 |
| 3.2 | 43.3 (110) | 94 | 7.0 | 99.3 | 49 |
| 2.5 | 43.8 (111) | 61 | 9.0 | 99.4 | 54 |
| 2.9 | 43.3 (110) | 316 | 2.3 | 97.8 | 51 |
| 3.0 | 42.7 (109) | 53 | 5.1 | 99.0 | 51 |
| 3.0 | 42.7 (109) | 99 | 5.4 | 99.1 | 49 |
| 4.8 | 42.7 (109) | 610 | 1.06 | 92.5 | 79 |

## Claims

1. A process for removing carbonyl sulfide from a liquid propylene feedstream to produce a propylene product containing less than about 50 wppb carbonyl sulfide and being substantially free of $CO_2$, $H_2O$ and $H_2S$ which comprises treating a liquid propylene feedstream containing water, the treatment comprising the steps of:

(a) conducting hydrolysis of the carbonyl sulfide in the liquid propylene feedstream by passing the feedstream over activated alumina in a hydrolysis reactor at a liquid hourly space velocity of about 1 - 10 cubic metre of feedstream per cubic metre of alumina and at a temperature of 15° to 93° C (60° to 200° F), the amount of water in the feedstream undergoing hydrolysis being such that the molar ratio of water to carbonyl sulfide is at least 15 to 1;

(b) continuing step (a) until the carbonyl sulfide content of the feedstream after hydrolysis is more than about 500 wppb, and in such event discontinuing hydrolysis by removing the hydrolysis reactor from service and regenerating the catalyst, said regeneration being conducted by passing an inert gas at 204° C - 260° C (400 - 500° F) through the catalyst for the period of 10 to 30 hours and thereafter resuming hydrolysis; and

(c) removing $CO_2$, $H_2O$, and $H_2S$ by adsorption and additional COS by supplemental hydrolysis and adsorption by passing the feedstream through one or more secondary reactors containing activated alumina operated at ambient temperature, whereby a liquid propylene containing less than 50 wppb COS and being substantially free of $CO_2$, $H_2O$ and $H_2S$ is obtained.

2. The process Claim 1 wherein the hydrolysis reactor is at a temperature of 35° to 43.3° C (95° to 110 °F).

3. The process of Claim 1 or 2 wherein the polypropylene feedstream contains water in an amount such that the molar ratio of water to carbonyl sulfide in the feedstream undergoing hydrolysis in step (a) is 50 to 1 to 300 to 1.

4. The process of Claim 1, 2 or 3 wherein step (c) employs two secondary reactors.

5. The process of Claim 1, 2, 3 or 4 wherein the regeneration gas may be hydrogen, methane, nitrogen, natural gas or a hydrocarbon containing gas.

6. The process of Claim 5 wherein the regeneration gas is at a temperature of about 232° C (450° F) and the regeneration step is conducted for a period of 16 - 18 hours.

7. The process of any of Claims 1 - 6 where the activated alumina has a surface area of 250 - 400 $m^2/g$.

8. The process of any of Claims 1 - 7 wherein the liquid hourly space velocity of the feedstream is 2.5 to 4.0.

## Patentansprüche

1. Verfahren zur Abtrennung von Carbonylsulfid aus einem flüssigen Propylen-Beschickungsstrom zur Herstellung eines Propylen-Produkts, welches weniger als etwa 50 wppb Carbonylsulfid enthält und im wesentlichen frei von $CO_2$, $H_2O$ und $H_2S$ ist, umfassend die Behandlung eines flüssigen, Wasser enthaltenden Propylen-Beschickungsstroms nach folgenden Schritten:

a) die Hydrolyse des Carbonylsulfids in dem flüssigen Propylen-Beschickungsstrom durchführen, indem der Beschickungsstrom in einem Hydrolyse-Reaktor über aktiviertes Aluminiumoxid bei einer flüssigen stündlichen Raumgeschwindigkeit von etwa 1 - 10 Kubikmeter Beschickungsstrom pro Kubikmeter Aluminiumoxid und bei einer Temperatur von 15° bis 93° C (60 bis 200° F) geleitet wird, und wobei die Menge an Wasser in dem Beschickungsstrom, welcher der Hydrolyse unterworfen wird, eine solche ist, dass das molare Verhältnis von Wasser zu Carbonylsulfid wenigstens 15 zu 1 ist;

b) Schritt a) fortführen, bis der Carbonylsulfidgehalt im Beschickungsstrom nach der Hydrolyse höher als etwa 500 wppb ist, und in diesem Fall die Hydrolyse unterbrechen, indem der Hydrolysereaktor abgeschaltet wird und der Katalysator regeneriert wird, wobei die Regenerierung durchgeführt wird, indem man ein Inertgas bei 204° bis 260° C (400 - 500° F) für einen Zeitraum von 10 bis 30 Stunden durch den Katalysator leitet und danach die Hydrolyse wieder aufnimmt, und

c) $CO_2$, $H_2O$ und $H_2S$ durch Adsorption und weiteres COS durch zusätzliche Hydrolyse und Adsorption entfernen, indem der Beschickungsstrom durch einen oder mehrere sekundäre Reaktoren geleitet wird, welcher aktiviertes Aluminiumoxid enthält und bei Raumtemperatur betrieben wird, wobei flüssiges Propylen erhalten wird, welches weniger als 50 wppb COS enthält und im wesentlichen frei von $CO_2$, $H_2O$ und $H_2S$ ist.

2. Verfahren gemäss Anspruch 1, wobei der Hydrolysereaktor bei einer Temperatur von 35° bis 43,3° C (95° bis 110° F) ist.

3. Verfahren gemäss einem der Ansprüche 1 oder 2, wobei der Polypropylen-Beschickungsstrom Wasser in einer solchen Menge enthält, dass das molare Verhältnis von Wasser zu Carbonylsulfid im Beschickungsstrom, welcher der Hydrolyse in Schritt a) unterworfen wird, 50 zu 1 bis 300 zu 1 beträgt.

4. Verfahren gemäss einem der Ansprüche 1, 2 oder 3, wobei der Schritt c) zwei Sekundarreaktoren umfasst.

5. Verfahren gemäss einem der Ansprüche 1, 2, 3 oder 4, worin das Regenerierungsgas Wasserstoff, Methan, Stickstoff, Erdgas oder ein Kohlenwasserstoff enthaltendes Gas ist.

6. Verfahren gemäss Anspruch 5, worin das Regenerierungsgas eine Temperatur von etwa 232° C (450° F) aufweist und der Regenerierungsschritt für einen Zeitraum von 16 - 18 Stunden durchgeführt wird.

7. Verfahren gemäss einem der Ansprüche 1 - 6, wobei das aktivierte Aluminiumoxid eine Oberfläche von 250

- 400 $m^2$/g hat.

8.Verfahren gemäss einem der Ansprüche 1 - 7, wobei die flüssige stündliche Raumgeschwindigkeit des Beschickungsstroms 2,5 bis 4,0 beträgt.

**Revendications**

1. Procédé pour éliminer le sulfure de carbonyle d'un courant de propylène liquide d'alimentation afin d'obtenir comme produit un propylène contenant moins d'environ 50 ppbp de sulfure de carbonyle et étant pratiquement dépourvu de $CO_2$, $H_2O$ et $H_2S$, qui consiste à traiter un courant de propylène liquide d'alimentation contenant de l'eau, le traitement comprenant les étapes consistant:

(a) à effectuer l'hydrolyse du sulfure de carbonyle dans le courant de propylène liquide d'alimentation en faisant passer le courant d'alimentation sur de l'alumine activée dans un réacteur d'hydrolyse à une vitesse spatiale liquide horaire de 1 à 10 mètres cubes de courant d'alimentation par mètre cube d'alumine et à une température de 15° à 93°C (60° à 200°F), la quantité d'eau dans le courant d'alimentation subissant une hydrolyse étant telle que le rapport molaire de l'eau au sulfure de carbonyle soit d'au moins 15 à 1;

(b) à poursuivre la mise en oeuvre de l'étape (a) jusqu'à ce que la teneur en sulfure de carbonyle du courant d'alimentation, après hydrolyse, soit supérieure à environ 500 ppbp et, dans un tel cas, à interrompre l'hydrolyse en mettant hors service le réacteur d'hydrolyse et en régénérant le catalyseur, ladite régénération étant effectuée en faisant passer un gaz inerte à une température de 204 à 260°C (400 à 500°F) à travers le catalyseur pendant un temps de 10 à 30 heures, puis à remettre en oeuvre l'hydrolyse; et

(c) à éliminer $CO_2$, $H_2O$ et $H_2S$ par adsorption, et une quantité supplémentaire de COS par une hydrolyse supplémentaire et par adsorption, en faisant passer le courant d'alimentation à travers un ou plusieurs réacteurs secondaires contenant de l'alumine activée, fonctionnant à température ambiante, un propylène liquide contenant moins de 50 ppbp de COS et étant pratiquement dépourvu de $CO_2$, $H_2O$ et $H_2S$ étant ainsi obtenu.

2. Procédé suivant la revendication 1, dans lequel le réacteur d'hydrolyse est à une température de 35$^c$ à 43,3°C (95° à 110°F).

3. Procédé suivant la revendication 1 ou 2, dans lequel le courant de polypropylène d'alimentation contient de l'eau en une quantité telle que le rapport molaire de l'eau au sulfure de carbonyle dans le courant d'alimentation subissant une hydrolyse dans l'étape (a) soit compris dans l'intervalle de 50 : 1 à 300 : 1.

4. Procédé suivant la revendication 1, 2 ou 3, dans lequel l'étape (c) utilise deux réacteurs secondaires.

5. Procédé suivant la revendication 1, 2, 3 ou 4, dans lequel le gaz de régénération peut être l'hydrogène, le méthane, l'azote, du gaz naturel ou un gaz contenant un hydrocarbure.

6. Procédé suivant la revendication 5, dans lequel le gaz de régénération est à une température d'environ 232°C (450°F) et l'étape de régénération est mise en oeuvre pendant un temps de 16 à 18 heures.

7. Procédé suivant l'une quelconque des revendications 1 à 6, dans lequel l'alumine activée possède une surface spécifique de 250 à 400 $m^2$/g.

8. Procédé suivant l'une quelconque des revendications 1 à 7, dans lequel la vitesse spatiale liquide horaire de la charge d'alimentation est comprise dans l'intervalle de 2,5 à 4,0.